# EUROPEAN PATENT SPECIFICATION

(11) **EP 1 405 912 B1**
(45) Date of publication and mention of the grant of the patent: **05.09.2012**
(21) Application number: 02745822.3
(22) Date of filing: 04.07.2002
(51) Int. Cl.: C12P 21/02

(54) **GENETICALLY MODIFIED ECARIN AND PROCESS FOR PRODUCING THE SAME**
GENETISCH MODIFIZIERTES ECARIN UND VERFAHREN ZU DESSEN HERSTELLUNG
ECARINE GENETIQUEMENT MODIFIEE ET PROCEDE D'ELABORATION

(30) Priority: 06.07.2001 JP 2001206918
(43) Date of publication of application: 07.04.2004
(73) Proprietor: Juridical Foundation The Chemo-Sero-Therapeutic Research Institute, Kumamoto-shi, Kumamoto 860-8568 (JP)
(72) Inventor: YONEMURA, Hiroshi, Kikuchi-gun, Kumamoto 869-1298 (JP); IMAMURA, Takayuki, Kikuchi-gun, Kumamoto 869-1298 (JP); NAKATAKE, Hiroshi, Kikuchi-gun, Kumamoto 869-1298 (JP); SOEJIMA, Kenji, Kikuchi-gun, Kumamoto 869-1298 (JP); NOZAKI, Chikateru, Kikuchi-gun, Kumamoto 869-1298 (JP)
(74) Representative: Vossius & Partner
(86) International application number: PCT/JP2002/006770
(87) International publication number: WO 2003/004647

(56) References cited:
- WO-A-01/04146
- US-A- 5 602 034
- HOFMANN H ET AL: "BLOOD COAGULATION INDUCED BY THE VENOM OF BOTHROPS ATROX. I. IDENTIFICATION, PURIFICATION AND PROPERTIES OF A PROTHROMBIN ACTIVATOR" BIOCHEMISTRY, AMERICAN CHEMICAL SOCIETY. EASTON, PA, US, vol. 26, no. 3, 1987, pages 772-780, XP001063236 ISSN: 0006-2960
- NOMURA M ET AL: "In vivo induction of cytotoxic T lymphocytes specific for a single epitope introduced into an unrelated molecule" JOURNAL OF IMMUNOLOGICAL METHODS, ELSEVIER SCIENCE PUBLISHERS B.V.,AMSTERDAM, NL, vol. 193, no. 1, 14 June 1996 (1996-06-14), pages 41-49, XP004020801 ISSN: 0022-1759
- RAKONJAC J ET AL: "Filamentous phage are released from the bacterial membrane by a two-step mechanism involving a short C-terminal fragment of pIII" JOURNAL OF MOLECULAR BIOLOGY, LONDON, GB, vol. 289, no. 5, 25 June 1999 (1999-06-25), pages 1253-1265, XP004451670 ISSN: 0022-2836
- NISHIDA S. ET AL.: 'cDNA cloning and deduced amino acid sequence of prothrombin activator (ecarin) from Kenyan Echis carinatus venom' BIOCHEMISTRY vol. 34, no. 5, 1995, pages 1771 - 1778, XP002159171
- MORITA T. ET AL.: 'Purification and properties of prothrombin activator from the venom of Echis carinatus' J. BIOCHEM. vol. 83, no. 2, 1978, pages 559 - 570, XP002955637
- BRIET E. ET AL.: 'Cleavage and activation of human prothrombin by Echis carinatus venom' THROMB. RES. vol. 27, no. 5, 1982, pages 591 - 600, XP002955638
- YAMAMOTO ET AL: JOURNAL OF CHROMATOGRAPHY, vol. 852, 1999, pages 37-41, XP004176667,

### SEQUENCE LISTING

<110> JURIDICAL FOUNDATION THE CHEMO-SERO-THERAPEUTIC RESEARCH INSTITUTE
<120> Recombinant echarine and method for preparing thereof
<130> 663289
<150> JP 2001-206918
   <151> 2001-07-06
<160> 5
<210> 1
   <211> 616
   <212> PRT
   <213> Echis carinatus
<400> 1
<210> 2
   <211> 1863
   <212> DNA
   <213> Echis carinatus
<400> 2
   ctcgagatga tccagattct cttggtaatt atatgcttag cagtttttcc atatcaaggt    60
   tgctctataa tcctgggatc tgggaatgtt aatgattatg aagtagtgta tccacaaaaa   120
   gtcactgcat tgcccaaagg agcagttcag cagcctgagc aaaagtatga agatgccatg   180
   caatatgaat ttgaagtgaa gggagagcca gtggtccttc acctagaaaa aaataaagaa   240
   cttttttcag aagattacag tgagactcat tattcgtctg atgacagaga aattacaaca   300
   aacccttcag ttgaggatca ctgctattat catggacgga tccagaatga tgctgagtca   360
   actgcaagca tcagtgcatg caatggtttg aaaggacatt tcaagcttcg aggggagacg   420
   tactttattg aacccttgaa gattcccgac agtgaagccc atgcagtcta caaatatgaa   480
   aacatagaaa atgaggatga agcccccaaa atgtgtgggg taacccagga taattgggaa   540
   tcagatgaac ccatcaaaaa gactttgggg ttaattgttc ctcctcatga acgaaaattt   600
   gagaaaaaat tcattgagct tgtcgtagtt gtggaccaca gtatggtcac aaaatacaac   660
   aatgattcaa ctgctataag aacatggata tatgaaatgc tcaacactgt aaatgagata   720
   tacttacctt tcaatattcg tgtagcactg gttggcctag aattttggtg caatggagac   780
   ttgattaacg tgacatccac agcagatgat actttgcact catttggaga atggagagca   840
   tcagatttgc tgaatcgaaa aagacatgat catgctcagt tactcacgaa cgtgacactg   900
   gatcattcca ctcttggaat cacgttcgta tatggcatgt gcaaatcaga tcgttctgta   960
   gaacttattc tggattacag caacataact tttaatatgg catatataat agcccatgag  1020
   atgggtcata gtctgggcat gttacatgac acaaaattct gtacttgtgg ggctaaacca  1080
   tgcattatgt ttggcaaaga aagcattcca ccgcccaaag aattcagcag ttgtagttat  1140
   gaccagtata acaagtatct tcttaaatat aacccaaaat gcattcttga tccacctttg 1200
   agaaaagata ttgcttcacc tgcagtttgt ggaaatgaaa tttgggagga aggagaagaa 1260
   tgtgattgtg gttctcctgc agattgtcga aatccatgct gtgatgctgc aacatgtaaa 1320
   ctgaaaccag gggcagaatg tggaaatgga gagtgttgtg acaagtgcaa gattaggaaa 1380
   gcaggaacag aatgccggcc agcaagggat gactgtgatg tcgctgaaca ctgcactggc 1440
   caatctgctg agtgtcccag aaatgagttc caaaggaatg gacaaccatg ccttaacaac 1500
   tcgggttatt gctacaatgg ggattgcccc atcatgttaa accaatgtat tgctctcttt 1560
   agtccaagtg caactgtggc tcaagattca tgttttcaga ggaacttgca aggcagttac 1620
   tatggctact gcacaaagga aattggttac tatggtaaaa ggtttccatg tgcaccacaa 1680
   gatgtaaaat gtggcagatt atactgctta gataattcat tcaaaaaaaa tatgcgttgc 1740
   aagaacgact attcatacgc ggatgaaaat aagggaatag ttgaacctgg aacaaaatgt 1800
   gaagatggaa aggtctgcat caacaggaag tgtgttgatg tgaatacagc ctactaactc 1860
   gag 1863
<210> 3
   <211> 31
   <212> DNA
   <213> Artificial Sequence
<220>
   <223> Primer
<400> 3
   atgcactcga gatgatccag attctcttgg t 31
<210> 4
   <211> 30
   <212> DNA
   <213> Artificial Sequence
<220>
   <223> Primer
<400> 4
   tgcatctcga gttagtaggc tgtattcaca 30
<210> 5
   <211> 20
   <212> PRT
   <213> Echis carinatus
<400> 5
   Lys Asn Asp Tyr Ser Tyr Ala Asp Glu Asn Lys Gly Ile Val Glu Pro
   5 10 15
   Gly Thr Lys Cys
   20

## Description

### TECHNICAL FIELD

The present invention relates to a process for preparing a recombinant ecarin comprising the steps as defined in the claims.

### BACKGROUND ART

Some animals have a potent venom such as a snake venom, a spider venom, a scorpion venom, and a bee venom. It has been revealed that the venom comprises practically useful substance including a neurotoxin, a bleeding toxin, a thrombotic toxin, a physiologically active peptide, a cell growth factor activity, and the like.

Ecarin is a snake venom-derived protease isolated from Echis carinatus (T. Morita et al.: J. Biochem. 83, 559-570, 1978), known to specifically activate prothrombin. A cDNA encoding ecarin has been cloned by S. Nishida et al. (Biochemistry, 34, 1771-1778, 1995) to reveal its structure. New forms of ecarin were described in WO 01/04146. Ecarin, a sugar protein, is a metalloprotease, a mature form of which has 426 amino acid residues in total, having a mosaic structure comprising a Zn²⁺ chelate, a disintegrin domain and a Cys-rich domain, with 61% homology to an H chain of RVV-X (Russell's viper venom X activator). Ecarin is quite a distinct enzyme from Factor Xa since its enzymatic activity is inactivated by EDTA but is not inhibited by DFP or antithrombin III.

Prothrombin, a target substrate to be activated by ecarin, is a precursor of thrombin, one of various blood coagulation factors. Thrombin, produced as a consequence of activation, is a multifunctional serine protease capable of interacting with various substrates and acting in a coagulation-anticoagulation manner within the living body. In human blood, prothrombin is activated by Factor Xa through restricted cleavage at two sites of prothrombin, i.e. Arg-Thr and Arg-Ile, whereas ecarin hydrolyzes prothrombin at the Arg-Ile alone to produce meizothrombin having a comparatively large molecular weight. Conversion of prethrombin-2 (product after removal of gla domain and Kringle domain from prothrombin) into active thrombin (α-thrombin) through Arg-Ile cleavage can surely be performed by ecarin based on its high substrate specificity. Although the Arg-Ile cleavage can also be done by trypsin, belonging to the same serine protease as ecarin, trypsin apt to cleave additional sites other than said cleavage site whereas ecarin never cleaves any other sites than said cleave site due to its high specificity as to the cleavage site. When trypsin is used to activate prethrombin-2, although partial activation of prethrombin-2 into α-thrombin is observed, most of the resulting α-thrombin is further degraded and hence complete conversion from prethrombin-2 into α-thrombin is not possible. On the contrary, when ecarin is added to prethrombin-2, conversion of prethrombin-2 into α-thrombin can quantitatively be done without any side products since ecarin only cleaves the unique Arg-Ile in prothrombin-2.

In the living process where prothrombin is converted into α-thrombin, prothrombin is activated via the intermediate called meizothrombin as described above. Within the living body, prothrombin is cleaved first at its Arg-Ile site by Factor Xa to produce meizothrombin, which is then cleaved by Factor Xa at the Arg-Thr site to complete activation to α-thrombin. Meizothrombin is known, though having a protease activity, to have a distinct substrate specificity from α-thrombin, as possessing an extremely low coagulating activity to fibrinogen while retaining an ability to activate protein C. Exhibiting such a characteristic substrate specificity, meizothrombin is readily converted into α-thrombin and hence does not stably exist within the living body since Factor Xa cleaves the two sites in prothrombin, i.e. Arg-Thr and Arg-Ile, as described above. Using ecarin for activation of prothrombin, however, meizothrombin can stably be prepared as ecarin cleaves the Arg-Ile site alone. Moreover, since ecarin also acts on abnormal prothrombin produced biosynthetically in the absence of Vitamin K, it is utilized for measuring blood level of such abnormal prothrombin.

### DISCLOSURE OF THE INVENTION

As explained above, ecarin was found to possess an industrially useful activity. Up till the present, however, the only source is Echis carinatus and thus ecarin has not yet been industrially utilized due to its limited quantitative availability. Under the present circumstances, ecarin is sold as a reagent by reagent manufacturers but, due to high price as well as insufficient provision, it is not possible to use ecarin for activating prethrombin-2 or for preparing meizothrombin on an industrially applicable large scale. Besides, there is no guarantee that wild Echis carinatus is stably provided as a source and safety of working staffs must also be taken care of in view of the snake venom who handle a breeding of the snake. Putting all these accounts together, one may readily find that obtaining a large amount of ecarin is much difficult. Taking into a consideration a risk and limitation in preparing ecarin from Echis carinatus, another source and method allowing for provision of ecarin in a safer and more stable manner is desired.

Under the circumstances, a problem to be solved by the present invention is, aiming to establish a process for efficiently preparing thrombin on an industrial scale, to provide ecarin by the genetic recombination technique as a means for thrombin production.

The present inventors have earnestly investigated to solve the above-mentioned problems and as a result have completed the present invention that provides a process as defined in the claims for efficiently preparing ecarin using the genetic recombination technique.

Thus, disclosed is the provision of a genetic recombinant ecarin (hereinafter also referred to as "recombinant ecarin") capable of serving conversion from prethrombin-2 into α-thrombin or from prothrombin into meizothrombin via cleavage at the Arg-Ile site. This is attained by providing a protein with a molecular weight of about 80,000 having an amino acid sequence as set forth in SEQ ID NO: 1, or a peptide fragment or a series of peptide fragments having said amino acid sequence with one or several amino acid residues therein being deleted, substituted or added, or a partial sequence of either of the above amino acid sequences, or an amino acid sequence comprising as a part any of the above amino acid sequences.

Also disclosed are a gene fragment that encodes the recombinant ecarin as described above and has a nucleotide sequence as set forth in SEQ ID NO: 2, or gene fragments that encode peptides having a partial amino acid sequence of said protein, as well as a plasmid comprising these gene fragments.

Also disclosed is a recombinant microorganism and animal cell transformed with said plasmid. The present invention encompasses a process for preparing a recombinant ecarin of interest or peptides having a partial amino acid sequence of the recombinant ecarin using these transformed microorganism or animal cells as defined in the claims.

The present invention relates to:
1. A process for preparing a recombinant ecarin which comprises the following steps:
   (1) culturing a transformant microorganism or animal cell transformed with an expression vector in which a gene encoding the protein of SEQ ID NO:1 is incorporated downstream of a promoter so as to produce and accumulate said protein in the culture supernatant or within said transformant and recovering the to produced protein;
   (2) adjusting the protein sample obtained in step (1) to pH 5.0 with citric acid;
   (3) applying said protein sample of step (2) to a cation exchange chromatography with a Macro-Prep High S Support column equilibrated with 20 mM citrate (pH 5.0) buffer;
   (4) washing the column with 20 mM citrate (pH 5.0) buffer;
   (5) eluting the protein with a gradient of salt concentration ranging from 0 mM to 1000 mM NaCl/20 mM citric acid (pH 5.0);
   (6) pooling fractions containing the protein and dialyzing against 20 mM sodium hydrogen carbonate buffer (pH 9.0) containing 50 mM NaCl;
   (7) subjecting the protein obtained in step (6) to cation exchange chromatography with a sulfate Cellulofine column equilibrated with 20 mM sodium hydrogen carbonate buffer (pH 9.0) containing 50 mM NaCl;
   (8) washing the column with 20 mM sodium hydrogen carbonate buffer (pH 9.0) containing 50 mM NaCl;
   (9) eluting the protein with a gradient of salt concentration ranging from 50 mM to 600 mM NaCl/20 mM sodium hydrogen carbonate (pH 9.0);
   (10) pooling fractions containing the protein; and
   (11) subjecting the purified protein obtained in step (10) to gel filtration chromatography to thereby remove impurities from a solution containing the recombinant ecarin.
2. The process of 1, wherein said promoter is selected from the group consisting of SV40 early promoter, SV40 late promoter, Cytomegalovirus promoter and chicken β-actin promoter.
3. The process of 2, wherein said promoter is chicken β-actin promoter.
4. The process of any one of 1 to 3, wherein said expression vector contains a signal sequence upstream of a gene encoding the protein of SEQ ID NO:1.
5. The process of 4, wherein said signal sequence is selected from the group consisting of pel B signal, α factor signal, immunoglobulin signal SG-1 and C25 signal.
6. The process of any one of 1 to 5, wherein said expression vector further contains a gene amplification gene, and the transformant is cultured under conditions suitable for gene amplification.
7. The process of 6, wherein said gene amplification gene is a gene encoding dihydrofolate reductase.
8. The process of any one of 1 to 7, wherein said gene encoding the protein of SEQ ID NO:1 is a gene fragment having the nucleotide sequence as set forth in SEQ ID NO:2 or a gene fragment encoding a peptide comprising a partial amino acid sequence of said protein.
9. The process of any one of 1 to 8, wherein said transformant is an animal cell selected from the group consisting of Chinese hamster ovary cell (CHO cell), mouse myeloma cell, BHK21 cell, 293 cell and COS cell.

### BRIEF DESCRIPTION OF DRAWINGS

Fig. 1 illustrates expression plasmid pCAGG-S1(Sal).dhhr.neo.
Fig. 2 shows the results of SDS-PAGE and protein staining for fractions obtained in gel filtration, the final step in ecarin purification from culture supernatant of ecarin-producing SP2/0.
Fig. 3 shows an activity to cleave S-2238 after addition of a recombinant ecarin to prothrombin.

### BEST MODE FOR CARRYING OUT THE INVENTION

A gene encoding ecarin protein can be obtained by PCR using the sequence reported in the literature (S. Nishida et al., Biochemistry, 34, p.1771-1778, 1995) as a template and synthetic DNAs as set forth in SEQ ID NO: 3 and SEQ ID NO: 4 as a primer pair.

Ecarin or a partial protein thereof can be prepared by incorporating a cDNA fragment encoding a portion of a structural region of a full length ecarin protein into an expression vector, transforming suitable microorganism or animal cells with the resulting expression vector, and culturing the transformant microorganism or animal cells to produce ecarin or a partial protein thereof. For production of a partial protein of ecarin, a peptide synthesizer may also be used.

An appropriate signal sequence for secretion in microorganism or animal cells may be linked to the upstream of a DNA encoding the protein of the present invention so that said protein may be expressed and secreted into a culture medium. The thus modified DNA for the purpose of secretion is advantageous in that the protein secreted into a culture medium can readily be recovered. Such a signal sequence includes pel B signal (S. P Lei et al., J. Bacteriology Vol. 169, 4379-4383, 1987) for E. coli; α factor signal (A. J. Brake, Yeast Genetic Engineering, p269 Butterworth, 1989) for yeast; immunoglobulin signal SG-1 (H. Maeda et al., Hum. Antibod. Hybridomas Vol. 2, 124-134, 1991), C25 signal (Patent, International Publication No. WO94/20632) for animal cells, and the like.

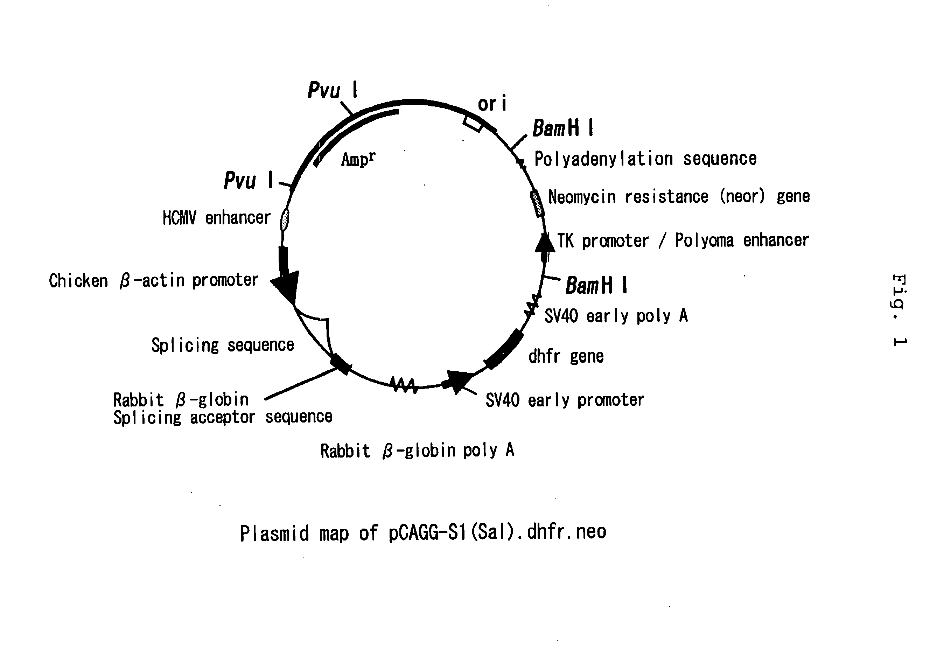

An expression vector that can be used includes a plasmid, a viral vector, and the like. A promoter to be contained in the expression vector may be any promoter as far as it can ultimately provide active ecarin protein when combined with microorganism or animal cells as a host cell, including SV40 early promoter, SV40 late promoter, Cytomegalovirus promoter, chicken β-actin promoter, and the like. A marker gene includes, in case of an expression vector for a microorganism, an ampicilin resistance gene, tetracycline resistance gene for E. coli as a host; Leu2 gene for yeast as a host, and the like. In case of an expression vector for an animal cell, aminoglycoside 3'phosphotransferase (neo) gene, dihydrofolate reductase (dhfr) gene, glutamine synthetase (GS) gene, and the like, may be used. Fig. 1 shows pCAGG-S1(Sal).dhhr.neo as a exemplary expression vector wherein substance to be added for selection includes G418, neomycin, methotrexate, and the like.

In case of an expression vector for an animal cell, a variety of cells such as Chinese hamster ovary (CHO) cell, mouse myeloma cell, e.g. SP2/0, BHK21 cell, 293 cell and COS cell may be used as a host cell. With the thus constructed ecarin expressing cells, ecarin may be expressed transiently using e.g. COS7 cell (derived from African green monkey) or expressed stably using SP2/0 cell or CHO cell as a host cell.

A host cell may be transformed by any known methods including, for example, a calcium phosphate method, a DEAE dextran method, precipitation with e.g. lipofectin, fusion of protoplast with polyethylene glycol, electroporation, and the like. A method for transformation may suitably be selected depending on a host cell as used.

The recombinant ecarin may be prepared as described below. The animal cells that stably express ecarin are cultured under normal conditions, for example, in MEM alpha culture medium containing 400 µg/mL to 1 mg/mL neomycin and 10% FCS in case that neomycin resistance gene is used as a marker gene to prepare neomycin resistant cells and then the enzymatic activity of ecarin in culture supernatant is measured. A clone that can grow under serum free conditions is then obtained from the ecarin producing transformants and cultured in a large scale. Ecarin is prepared and purified from the culture supernatant recovered therefrom with an index of the activity of ecarin to convert prothrombin into α-thrombin.

The recombinant ecarin may be isolated and purified by any method conventionally used in the field of protein chemistry, as appropriately selected, including e.g. a salting out, ultrafiltration, isoelectric focusing, electrophoresis, ion exchange chromatography, hydrophobic chromatography, gel filtration chromatography, reverse phase chromatography, affinity chromatography, etc., with an index of the ability to activate prothrombin as described above. A preferable embodiment for purifying the recombinant ecarin includes a cation exchange chromatography and a gel filtration chromatography in this order under conditions as described in Example 7. This step allows for purification of the recombinant ecarin protein to around 2,600-fold higher specific activity with an activity yield of 13% with an index of the ability to activate prothrombin.

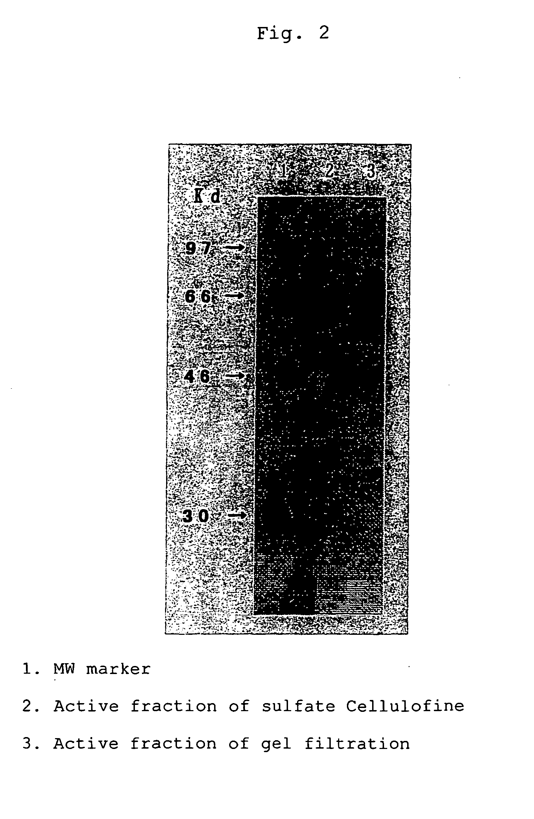

The fraction thus obtained after purification is then subject to sodium dodecyl sulfate polyacrylamide gel electrophoresis (SDS-PAGE) analysis in the presence of 2-mercaptoethanol to detect a main band of around 80,000 M.W. (Fig. 2).

The thus obtained ecarin has an activity to activate prothrombin. The ecarin protein may be utilized for preparing meizothrombin from prothrombin, for activating prethrombin-2 into α-thrombin, or for use as a detection reagent for abnormal prothrombin.

Besides, the ecarin protein or a polypeptide having a partial amino acid sequence thereof may also be utilized as an immunogen for preparing a polyclonal or monoclonal antibody by a conventional method. The thus prepared antibody capable of binding to the ecarin protein or a polypeptide having a partial amino acid sequence thereof as well as said protein or the polypeptide may be used in a detection system for an antigen such as Western blot or ELISA and as a material for constructing a diagnostic agent. Alternatively, the antibody may be bound to a suitable carrier for use in affinity chromatography to purify the antigenic proteins.

The present invention is explained in more detail by means of the following Examples which are not intended to restrict a scope of the present invention in any sense. Reagents used in the following Preparation and Examples were obtained from Pharmacia, BioRad, Wako Pure Chemical Industries, Ltd., TAKARA SHUZO CO., Ltd., Toyobo, and New England BioLabs.

### Example 1

### (Construction of expression plasmid)

### (1) Construction of expression plasmid pCAGG-S1(Sal)

A chicken β-actin promoter-based expression plasmid pCAGG (Japanese Patent Publication No. 168087/1991) was digested with restriction enzyme EcoRI, blunt ended with T4 DNA polymerase, and then ligated with T4 DNA ligase in the presence of phosphorylated XhoI linker to construct pCAGG(Xho). The obtained pCAGG(Xho) was digested with restriction enzyme SalI, blunt ended with T4 DNA polymerase, and then ligated with T4 DNA ligase to construct pCAGG-Pv2. The resulting pCAGG-Pv2 was digested with restriction enzyme XhoI and then treated with S1 nuclease to erase several nucleotides in the vicinity of the XhoI recognition site. After the nuclease treatment, a single chain region was modified with T4 DNA polymerase in the presence of dNTPs and then ligated with T4 DNA ligase in the presence of phosphorylated SalI linker to construct pCAGG-S1(Sal).

### (2) Construction of expression plasmid pCAGG-S1(Sal).dhfr

Expression plasmid pSV2-dhfr bearing DHFR gene (S. Subramani et al., Mol. Cell. Biol., 1, p.854-864, 1981) was digested with restriction enzyme BglII, blunt ended with T4 DNA polymerase, and ligated with T4 DNA ligase to construct pSV2-dhfr-Bgn. The resulting pSV2-dhfr-Bgn was then digested with restriction enzyme PvuII and ligated with T4 DNA ligase in the presence of phosphorylated BglII linker to construct pSV2-dhfr-BgB. The obtained pSV2-dhfr-BgB was digested with restriction enzymes BglII and BamHI and was then subject to agarose gel electrophoresis to obtain a fragment of about 1.7 kbp. The expression plasmid pCAGG-S1(Sal) obtained above was digested with restriction enzyme BamHI and then ligated to cyclize with the 1.7 kbp fragment to construct pCAGGS1(Sal).dhfr.

### (3) Construction of expression plasmid pCAGG-S1(Sal).dhfr.neo

An aminoglycoside phosphotransferase (neo^{r})-based expression plasmid pMClneo-polyA (K. R. Thomas et al., Cell, 51, p.503-512, 1987) was digested with restriction enzyme XhoI and then ligated with T4 DNA ligase in the presence of phosphorylated BamHI linker to construct pMClneo-2B. The resulting pMC1neo-2B was digested with restriction enzyme BamHI and then subject to agarose gel electrophoresis to obtain a fragment of about 1.1 kbp. The expression plasmid pCAGG-S1(Sal).dhfr obtained above was digested with restriction enzyme BamHI and then ligated to cyclize with the fragment of about 1.1 kbp to construct pCAGG-S1(Sal).dhfr.neo (Fig. 1).

### Example 2

### (Preparation of cDNA of snake venom ecarin)

Using the nucleotide sequence of ecarin cDNA reported in the literature (S. Nishida et al., Biochemistry, 34, p.1771-1778, 1995) as a template, PCR was conducted using a synthetic DNA having the sequence:
ATGCACTCGAGATGATCCAGATTCTCTTGGT (SEQ ID NO: 3)
and a synthetic DNA having the sequence
TGCATCTCGAGTTAGTAGGCTGTATTCACA (SEQ ID NO: 4)
as a primer pair to introduce the recognition sites of restriction enzyme XhoI at both termini. The obtained gene was digested with restriction enzyme XhoI and subcloned into pUC18 to construct pUC.EC. A nucleotide sequence of the ecarin cDNA region of the resulting plasmid was determined by the conventional method to thereby obtain ecarin cDNA that has an exactly identical nucleotide sequence from the initiation codon to the termination codon to the sequence reported in the literature (SEQ ID NO: 2).

The ecarin cDNA obtained herein encodes the polypeptide as set forth in SEQ ID NO: 1.

### Example 3

### (Construction of ecarin expression plasmid)

The ecarin cDNA obtained in Example 2 was incorporated into the expression vector pCAGG-S1(Sal).dhfr.neo obtained in Example 1. The plasmid pCAGG-S1(Sal).dhfr.neo was digested with restriction enzyme SalI and then dephosphorylated with bovine small intestine derived alkaline phosphatase. The plasmid pUC.EC obtained above was digested with restriction enzyme XhoI and then a fragment of about 1.8 kbp encoding ecarin cDNA was purified by agarose gel electrophoresis. Then, the dephosphorylated plasmid and the fragment encoding ecarin cDNA were ligated to cyclize with T4 DNA ligase to construct pCAGG-S1.EC.dhfr.neo.

### Example 4

### (Expression of ecarin using animal cells)

The ecarin expression plasmid pCAGG-S1.EC.dhfr.neo described in Example 3 was used to transform CHO cells and SP2/0 Ag14 cells. CHO cells were transformed by a modified calcium phosphate method whereas SP2/0-Ag14 cells were transformed by electroporation.

The expression plasmid for use in transformation was previously linearized by digestion with restriction enzyme PvuI.

### (1) Assessment of ability to produce ecarin with CHO cells

Using CHO cells, transformants were selected from transformation as described below.

On the day previous to transformation, the cells were plated in MEM alpha medium with nucleic acids supplemented with 10% fetal calf serum in 10 cm dish at a cellular density of 5 x 10⁵ cells/dish. After culture at 37°C overnight, the cells were transformed with 20 µg/mL of the linearized expression plasmid pCAGG-S1.EC.dhfr.neo. After culture in 3% CO₂ incubator at 35°C overnight, the cells were washed with Dulbecco PBS(-) and the culture medium was replaced with nucleic acid free MEM alpha medium containing 10% dialyzed FCS and 500 µg/mL Geneticin. For selection, culture was continued in 5% CO₂ incubator at 37°C while replacing the culture medium every 3 to 4 days and emerged transformants were pooled and assessed for their ability to produce ecarin.

The transformed cells were plated in nucleic acid free MEM alpha medium supplemented with 10% dialyzed FCS at a density of 2 x 10⁵ cells/mL and cultured overnight. The next day, the culture medium was replaced with serum free YMM medium (nucleic acid free MEM alpha medium with enriched amino acids/vitamins containing insulin, transferrin, ethanolamine and sodium selenite). After culture in 5% CO₂ incubator at 35°C for about 14 days, an ecarin level in culture supernatant was measured. As a result, 10 U/mL of ecarin in the culture supernatant was detected.

### (2) Assessment of ability to produce ecarin with SP2/0 cells

Using SP2/0 cells, transformants were selected from transformation as described below.

SP2/0 cells were washed twice with cooled Dulbecco PBS(-) and 10⁷ cells suspended in 0.8 mL of PBS(-) were placed in a cuvette for electroporation (electrode width 0.4 cm; manufactured by BIO-RAD). The linearized expression plasmid (40 µg) was added and mixed with pipette. One pulse was applied at 0.22 kv at 975 µF using Gene Pulser II (manufactured by BIO-RAD). After the cuvette was cooled on ice for 10 minutes, the cell suspension was diluted with MEM alpha medium with nucleic acids containing 10% fetal calf serum (FCS) to about 5,000 cells/50 µL, plated on five 96-well plates each at 50 µL/well, and cultured in 3% CO₂ incubator at 35°C overnight. The next day, 50 µL/well of nucleic acid free MEM alpha medium containing 10% dialyzed FCS was added and culture was further continued overnight. The next day, 100 µL/well of nucleic acid free MEM alpha medium containing 1 mg/mL Geneticin and 10% dialyzed FCS was added. After culture for 10 to 14 days, emerged transformants at each well were assessed for their ability to produce ecarin. The cells were plated with nucleic acid free MEM alpha medium containing 500 µg/mL Geneticin and 2% dialyzed FCS at a density of about 3 x 10⁵ cells/mL. After culture for about 14 days, an ecarin level in culture supernatant was measured. As a result, each of the transformants was found to express 2 to 10 U/mL of ecarin. Among these transformants, each 200 µL/well of those producing a high level ecarin were plated on 96-well plate at a concentration of 0.5 cell/well with the same culture medium for cloning by limiting dilution. Each of the obtained clones was assessed for their ability to produce ecarin. Each clone was plated with nucleic acid free MEM alpha medium containing 2% dialyzed FCS at a density of 3 x 10⁵ cells/mL. After culture in 5% CO₂ incubator at 35°C for about 14 days, an ecarin level in culture supernatant was measured. Among the obtained clones, clone #1H-8 expressed 15 U/mL ecarin in culture supernatant.

This clone #1H-8 was adapted to serum free medium using YMM medium. YMM medium with 2% dialyzed FCS was used for culture and growth of the cells was confirmed. Thereafter, culture was continued while the serum level to be added was gradually lowered to 0.5% and further to 0.1%. After the cells were confirmed to proliferate well, they were cultured with completely serum free YMM medium. Growth of the cells was confirmed and then their ability to produce ecarin was assessed by the method described above using YMM medium. The clone #1H-8 after adaptation to serum free culture possessed an ability to produce 20 U/mL ecarin.

### Example 5

### (Large scale culture of ecarin producing cells)

The ecarin producing cells #1H-8, adapted to serum free culture as described in Example 4, were subject to suspension culture with a spinner flask. After expansion of the cells, 250 mL of the cells were cultured with YMM medium in a 250 mL spinner flask (manufactured by Techne) at a density of 2 x 10⁵ cells/mL. The cells were expanded to a 1 L spinner flask at a density of more than 1 x 10⁶ cells/mL. After growth of the cells was confirmed, the cells were further expanded to five 1 L spinner flasks. The cells were cultured for about 7 days and then an ecarin level in culture supernatant was measured to detect expression of about 18 U/mL ecarin.

### Example 6

### (Preparation of antibody against partial peptide of ecarin)

An amino acid sequence encoded by the ecarin cDNA was analyzed for its hydrophilic and hydrophobic regions in accordance with Hopp and Wood (T. P. Hopp et al., Proc. Natl. Acad. Sci. Vol. 78, 3824-3828, 1981). As a high hydrophilicity region, a peptide having the amino acid sequence: Lys-Asn-Asp-Tyr-Ser-Tyr-Ala-Asp-Glu-Asn-Lys-Gly-Ile-Val-Glu-Pro-Gly-Thr-Lys-Cys as set forth in SEQ ID NO: 5 was synthesized with a peptide synthesizer (manufactured by Applied). This peptide (500 µg) was inoculated to rabbit intradermally in the presence of Freund complete adjuvant on Day 0 and in the presence of Freund incomplete adjuvant on Day 14 and Day 28 to prepare a polyclonal antibody against the ecarin peptide. Western blot was used to confirm whether the obtained antibody recognizes ecarin. Natural ecarin was subject to SDS-PAGE in the absence of 2-mercaptoethanol. After electrophoresis, the gel was immersed in a transfer buffer (10 mM N-cyclohexyl-3-aminopropanesulfonic acid, 10% methanol, pH 11) for 5 minutes and then overlayed on PVDF membrane (Immovilon: Millipore) previously immersed in 100% methanol and the transfer buffer in this order to perform transfer at 160 mA for 16 hours using TRANS-BLOTCELL (BIO-RAD). After masking with TBST (50 mM Tris-HCl, pH 8.0; 150 mM NaCl; 0.05% Tween 20, containing 5% skim milk), the membrane was incubated with the serum diluted by 500-fold with TBST from rabbit to which the synthetic peptide was administered at room temperature for 1 hour and then washed with TBST. Then, the membrane was reacted with anti-rabbit IgG-HRP labeled antibody (Bio-Rad) diluted by 2,000-fold at room temperature for 1 hour. After washing, the membrane was dyed with Konica Immunostaining HRP 1000 (Konica) kit. As a result, the serum obtained by immunization with the synthetic peptide proved to specifically react with ecarin.

### Example 7

### (Purification of ecarin)

### (1) Cation exchange chromatography

Culture supernatant (2000 mL) from the ecarin producing SP2/0 cells was diluted with a twice amount of water, adjusted to pH 5.0 with 1M citric acid and filtered through 0.45 µm filter to be used as a sample. The sample was applied to Macro-Prep High S Support (20 mL: Bio-Rad Laboratories) column equilibrated with 20 mM citrate (pH 5.0) buffer at a flow rate of 4 mL/min. The column was washed with the same buffer (150 mL) and then eluted with a gradient of salt concentration ranging from 0 mM to 1000 mM NaCl/20 mM citric acid (pH 5.0; 210 mL) at a flow rate of 4 mL/min. A portion of fractions was used for Western blot with the anti-ecarin antibody obtained in Example 6 to identify fractions with eluted ecarin, which were pooled and dialyzed against 20 mM sodium hydrogen carbonate buffer (pH 9.0) containing 50 mM NaCl.

### (2) Cation exchange chromatography

The dialyzed product obtained in the process of cation exchange chromatography (1) above was applied to sulfate Cellulofine (2 mL: SEIKAGAKU CORPORATION) column equilibrated with 20 mM sodium hydrogen carbonate buffer (pH 9.0) containing 50 mM NaCl at a flow rate of 0.5 mL/min. The column was washed with the buffer described above (14 mL) and then eluted with a gradient of salt concentration ranging from 50 mM to 600 mM NaCl/20 mM sodium hydrogen carbonate (pH 9.0; 20 mL) at a flow rate of 0.5 mL/min. A portion of fractions was used for Western blot with the anti-ecarin antibody obtained in Example 6 to identify and pool fractions with eluted ecarin.

### (3) Gel filtration

The fractions containing the recombinant ecarin obtained in the process of chromatography (2) above were applied to gel filtration column HiLoad 16/60 (Pharmacia) equilibrated with 10 mM phosphate (pH 7.0) buffer containing 100 mM NaCl and fractionated at a flow rate of 0.5 mL/min. A marker for gel filtration (Bio Rad) was used as a molecular weight standard. Each of the fractions was measured for an ability to activate prothrombin to detect a peak activity in a fraction of about M.W. 80,000. The obtained fraction of purified ecarin was subject to SDS-PAGE in the presence of 2-mercaptoethanol with subsequent treatment with Coomassie Brilliant Blue. The obtained pattern is shown in Fig. 2.

The above three-step purification gave the recombinant ecarin with 13% of final activity yield and 2,600-fold higher specific activity as compared to culture supernatant.

### Example 8

### (Activation of prothrombin by recombinant ecarin)

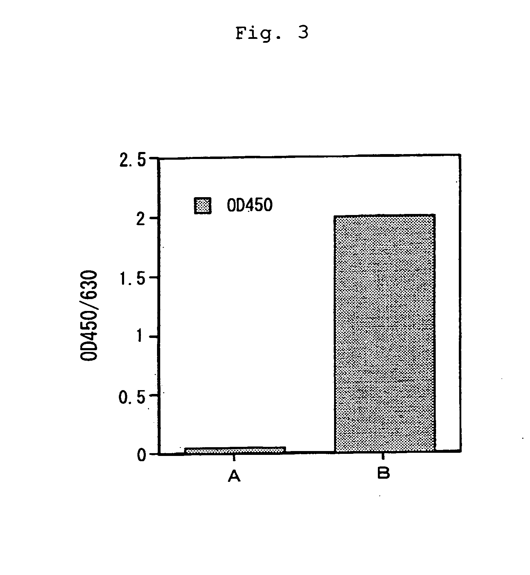

To prothrombin (20 mM Tris-HCl, 100 mM NaCl, pH 8.5; 1 mg/mL; 40 mL) was added benzamidine to a final concentration of 50 mM. The recombinant ecarin was added to the mixture to a final concentration of 2 U/mL. The reaction mixture was incubated at 37°C for 16 hours and then determined for the enzymatic activity of thrombin in accordance with the method as described below. As a result, the activity to cleave S-2238 was detected in prothrombin when added with the recombinant ecarin as shown in Fig. 3.

### Example 9

### (N-terminal amino acid sequence of prothrombin B chain activated by recombinant ecarin)

It is known that ecarin specifically cleaves the peptide bond in prothrombin at the Arg-Ile site to produce A chain and B chain from prothrombin. The N-terminal amino acid sequence of thrombin B chain obtained after activation in Example 8 was determined.

The sample was run on SDS-PAGE gel containing 15% polyacrylamide with 2-mercaptoethanol treatment. After electrophoresis, the gel was immersed in a transfer buffer (10 mM N-cyclohexyl-3-aminopropanesulfonic acid, 10% methanol, pH 11) for 5 minutes and then overlayed on PVDF membrane (Immovilon: Millipore) previously immersed in 100% methanol and the transfer buffer in this order to perform transfer at 160 mA for 16 hours using TRANS-BLOTCELL (BIORAD). The PVDF membrane after transfer was washed with water, dyed with 0.1% Amide Black (containing 40% methanol, 1% citric acid) for 1 minute and then decolorized with distilled water. A dyed band corresponding to the molecular weight of B chain was cut and the membrane fragment was analyzed with 477A Protein Sequencer (Applied Biosystems). A sequence of ten amino acid residues at the N-terminus was determined: Ile-Val-Glu-Gly-Ser-Asp-Ala-Glu-Ile-Gly. This sequence is identical to the N-terminal amino acid sequence of B chain of α-thrombin derived from human blood, demonstrating that the recombinant ecarin obtained according to the present invention specifically cleaves the peptide bond at the Arg-Ile site in prothrombin like snake venom derived ecarin.

Measurement of activity of thrombin and ecarin in Examples as described above was performed as follows:

### (1) Measurement of thrombin activity

An activity of thrombin was measured as described below.

A sample (20 µL), 50 mM Tris-HCl, pH 8.5 plus 50 mM NaCl buffer (60 µL), and 0.1% PLURONIC F-68 (20 µL) were added to 2008 tube (Falcon) and incubated at 37°C for 3 minutes. A purified α-thrombin derived from human plasma (purchased from Hematologic Technology: HCT-0020) was used as a standard with dilution to 5, 2.5, 1.25, 0.625, and 0.3125 µg/mL using the same buffer. To the reaction mixture was added 100 µL of TestTeam developing substrate S-2238 (1 mM: DAIICH PURE CHEMICALS CO., LTD.) while stirring. After reaction at 37°C for 5 minutes, the reaction was quenched with 800 µL of 0.1 M citric acid. The reaction solution (200 µL) was transferred to a 96-well plate and OD 405/650 was measured.

### (2) Measurement of ecarin activity

An activity of ecarin was measured as described below.

A sample (20 µL), and 50 mM Tris-HCl, pH 8.5 plus 50 mM NaCl plus 0.1% PLURONIC F-68 buffer ("Buffer 1"; 60 µL) were added to 2008 tube (Falcon). Thereto was added 0.01% trypsin (2 µL) and the mixture was stirred and incubated at 37°C for 10 minutes. The sample was diluted with Buffer 1 as needed depending on its concentration. To the reaction solution was added 10 µL of prothrombin (0.4 mg/mL; purchased from Hematologic Technology) and the mixture was reacted at 37°C for 5 minutes. Then, 10 mM EDTA (10 µL) and TestTeam developing substrate S-2238 (1 mM; 100 µL) were added to the reaction mixture while stirring. After reaction at 37°C for 5 minutes, the reaction was quenched with 800 µL of 0.1 M citric acid. The reaction solution (200 µL) was transferred to a 96-well plate and OD 405/650 was measured. For quantification of an ecarin activity, ecarin derived from snake venom (commercially available from Sigma) was diluted to 25 mU/mL, 12.5, 6.25, and 3.125 mU/mL with Buffer 1. Each 20 µL of these standard solutions was used in place of the sample without addition of the trypsin solution and the steps described above following the addition of prothrombin were repeated.

In accordance with the process of the present invention, the recombinant ecarin protein that specifically activates prothrombin can be provided. The recombinant ecarin prepared by the present inventors as described herein is a novel protein not previously reported. Thus, the technical problem is solved by the present invention by a process for preparing a recombinant ecarin, thus allowing for preparation of said protein on industrial scale.

## Claims

1. A process for preparing a recombinant ecarin which comprises the following steps:
(1) culturing a transformant microorganism or animal cell transformed with an expression vector in which a gene encoding the protein of SEQ ID NO:1 is incorporated downstream of a promoter so as to produce and accumulate said protein in the culture supernatant or within said transformant and recovering the so produced protein;
(2) adjusting the protein sample obtained in step (1) to pH 5.0 with citric acid;
(3) applying said protein sample of step (2) to a cation exchange chromatography with a Macro-Prep High S Support column equilibrated with 20 mM citrate (pH 5.0) buffer;
(4) washing the column with 20 mM citrate (pH 5.0) buffer;
(5) eluting the protein with a gradient of salt concentration ranging from 0 mM to 1000 mM NaCl/20 mM citric acid (pH 5.0);
(6) pooling fractions containing the protein and dialyzing against 20 mM sodium hydrogen carbonate buffer (pH 9.0) containing 50 mM NaCl;
(7) subjecting the protein obtained in step (6) to cation exchange chromatography with a sulfate Cellulofine column equilibrated with 20 mM sodium hydrogen carbonate buffer (pH 9.0) containing 50 mM NaCl;
(8) washing the column with 20 mM sodium hydrogen carbonate buffer (pH 9.0) containing 50 mM NaCl;
(9) eluting the protein with a gradient of salt concentration ranging from 50 mM to 600 mM NaCl/20 mM sodium hydrogen carbonate (pH 9.0);
(10) pooling fractions containing the protein; and
(11) subjecting the purified protein obtained in step (10) to gel filtration chromatography to thereby remove impurities from a solution containing the recombinant ecarin.

2. The process of claim 1, wherein said promoter is selected from the group consisting of SV40 early promoter, SV40 late promoter, Cytomegalovirus promoter and chicken β-actin promoter.

3. The process of claim 2, wherein said promoter is chicken β-actin promoter.

4. The process of any one of claims 1 to 3, wherein said expression vector contains a signal sequence upstream of a gene encoding the protein of SEQ ID NO:1.

5. The process of claim 4, wherein said signal sequence is selected from the group consisting of pel B signal, α factor signal, immunoglobulin signal SG-1 and C25 signal.

6. The process of any one of claims 1 to 5, wherein said expression vector further contains a gene amplification gene and the transformant is cultured under conditions suitable for gene amplification.

7. The process of claim 6, wherein said gene amplification gene is a gene encoding dihydrofolate reductase.

8. The process of any one of claims 1 to 7, wherein said gene encoding the protein of SEQ ID NO:1 is a gene fragment having the nucleotide sequence as set forth in SEQ ID NO:2 or a gene fragment encoding a peptide comprising a partial amino acid sequence of said protein.

9. The process of any one of claims 1 to 8, wherein said transformant is an animal cell selected from the group consisting of Chinese hamster ovary cell (CHO cell), mouse myeloma cell, BHK21 cell, 293 cell and COS cell.

## Patentansprüche

1. Verfahren zur Herstellung eines rekombinanten Ecarins, das die folgenden Schritte umfasst:
(1) das Züchten eines transformierten Mikroorganismus oder einer tierischen Zelle, die mit einem Expressionsvektor transformiert ist, in den ein Gen, das das Protein der SEQ ID NO:1 codiert, stromabwärts eines Promotors eingebracht ist, so dass das Protein hergestellt und im Kulturüberstand oder innerhalb der transformierten Zelle angereichert wird, und das Gewinnen des derart hergestellten Proteins;
(2) das Einstellen der Proteinprobe, die in Schritt (1) erhalten wurde, mit Citronensäure auf pH 5.0;
(3) das Auftragen der Proteinprobe aus Schritt (2) auf eine Kationenaustauscherchromatographie mit einer Macro-Prep High S Support-Säule, die mit 20 mM Citratpuffer (pH 5.0) äquilibriert ist;
(4) das Waschen der Säule mit 20mM Citratpuffer (pH 5.0);
(5) das Eluieren des Proteins mit einem Salzkonzentration-Gradienten, der sich von 0 mM bis 1000 mM NaCl/20 mM Citronensäure (pH 5.0) erstreckt;
(6) das Vereinigen von Fraktionen, die das Protein enthalten, und das Dialysieren gegen 20 mM Natriumhydrogencarbonat-Puffer (pH 9.0), der 50mM NaCl enthält;
(7) das Auftragen des Proteins, das in Schritt (6) erhalten wurde, auf eine Kationenaustauscherchromatographie mit einer Sulfat-Cellulofine-Säule, die mit 20 mM Natriumhydrogencarbonat-Puffer (pH 9.0), der 50mM NaCl enthält, äquilibriert ist;
(8) das Waschen der Säule mit 20 mM Natriumhydrogencarbonat-Puffer (pH 9.0), der 50mM NaCl enthält;
(9) das Eluieren des Proteins mit einem Salzkonzentration-Gradienten, der sich von 50 mM bis 600 mM NaCl/20 mM Natriumhydrogencarbonat (pH 9.0) erstreckt;
(10) das Vereinigen der Fraktionen, die das Protein enthalten; und
(11) das Auftragen des gereinigten Proteins, das in Schritt (10) erhalten wurde, auf eine Gelfiltrationschromatographie, um damit Verunreinigungen aus einer Lösung, die das rekombinante Ecarin enthält, zu entfernen.

2. Verfahren nach Anspruch 1, wobei der Promotor ausgewählt ist aus der Gruppe bestehend aus frühem SV40-Promotor, spätem SV40-Promotor, Cytomegalievirus-Promotor und β-Actin-Promotor vom Huhn.

3. Verfahren nach Anspruch 2, wobei der Promotor der β-Actin-Promotor vom Huhn ist.

4. Verfahren nach einem der Ansprüche 1 bis 3, wobei der Expressionsvektor eine Signalsequenz stromaufwärts eines Gens enthält, das das Protein der SEQ ID NO:1 codiert.

5. Verfahren nach Anspruch 4, wobei die Signalsequenz ausgewählt ist aus der Gruppe bestehend aus pel B-Signal, α-Faktor-Signal, Immunglobulin-Signal SG-1 und C25-Signal.

6. Verfahren nach einem der Ansprüche 1 bis 5, wobei der Expressionsvektor zudem ein Genamplifikationsgen enthält und die Transformante unter Bedingungen gezüchtet wird, die für die Genamplifikation geeignet sind.

7. Verfahren nach Anspruch 6, wobei das Genamplifikationsgen ein Gen ist, das Dihydrofolatreduktase codiert.

8. Verfahren nach einem der Ansprüche 1 bis 7, wobei das Gen, das das Protein der SEQ ID NO:1 codiert, ein Genfragment ist, das die wie in SEQ ID NO:2 dargestellte Nucleotidsequenz hat, oder ein Genfragment, das ein Peptid codiert, das eine Teilaminosäuresequenz des Proteins enthält.

9. Verfahren nach einem der Ansprüche 1 bis 8, wobei die Transformante eine tierische Zelle ist, die ausgewählt ist aus der Gruppe bestehend aus einer Ovarialzelle des Chinesischen Hamsters (CHO-Zelle), Maus-Myelomzelle, BHK21-Zelle, 293-Zelle und COS-Zelle.

## Revendications

1. Procédé pour préparer une écarine recombinante qui comprend les étapes suivantes :
(1) cultiver un micro-organisme transformant ou une cellule animale transformée avec un vecteur d'expression où un gène codant la protéine de SEQ ID NO:1 est incorporé en aval d'un promoteur de manière à produire et accumuler ladite protéine dans le surnageant de culture ou à l'intérieur dudit transformant et récupérer la protéine ainsi produite ;
(2) ajuster l'échantillon de protéine obtenu dans l'étape (1) à pH 5,0 avec de l'acide citrique ;
(3) appliquer ledit échantillon de protéine de l'étape (2) à une chromatographie d'échange de cations avec une colonne Macro-Prep High S Support équilibrée avec du tampon citrate (pH 5,0) 20 mM ;
(4) laver la colonne avec du tampon citrate (pH 5,0) 20 mM ;
(5) éluer la protéine avec un gradient de concentration de sel allant de 0 mM à 1 000 mM de NaCl/20 mM d'acide citrique (pH 5,0) ;
(6) regrouper les fractions contenant la protéine et dialyser contre du tampon hydrogénocarbonate de sodium 20 mM (pH 9,0) contenant 50 mM de NaCl ;
(7) soumettre la protéine obtenue dans l'étape (6) à une chromatographie d'échange de cations avec une colonne Cellulofine sulfate équilibrée avec du tampon hydrogénocarbonate de sodium (pH 9,0) 20 mM contenant 50 mM de NaCl ;
(8) laver la colonne avec du tampon hydrogénocarbonate de sodium (pH 9,0) 20 mM contenant 50 mM de NaCl ;
(9) éluer la protéine avec un gradient de concentration de sel allant de 50 mM à 600 mM de NaCl/20 mM d'hydrogénocarbonate de sodium (pH 9,0) ;
(10) regrouper les fractions contenant la protéine ; et
(11) soumettre la protéine purifiée obtenue dans l'étape (10) à une chromatographie de gel filtration pour retirer ainsi les impuretés d'une solution contenant l'écarine recombinante.

2. Procédé selon la revendication 1, où ledit promoteur est choisi dans le groupe consistant en le promoteur précoce de SV40, le promoteur tardif de SV40, le promoteur du Cytomégalovirus et le promoteur de β-actine de poulet.

3. Procédé selon la revendication 2, où ledit promoteur est le promoteur de β-actine de poulet.

4. Procédé selon l'une quelconque des revendications 1 à 3, où ledit vecteur d'expression contient une séquence signal en amont d'un gène codant la protéine de SEQ ID NO:1.

5. Procédé selon la revendication 4, où ladite séquence signal est choisie dans le groupe consistant en le signal pel B, le signal de facteur α, le signal d'immunoglobuline SG-1 et le signal C25.

6. Procédé selon l'une quelconque des revendications 1 à 5, où ledit vecteur d'expression contient en outre un gène d'amplification génique, et le transformant est cultivé dans des conditions appropriées pour l'amplification génique.

7. Procédé selon la revendication 6, où ledit gène d'amplification génique est un gène codant la dihydrofolate réductase.

8. Procédé selon l'une quelconque des revendications 1 à 7, où ledit gène codant la protéine de SEQ ID NO:1 est un fragment de gène ayant la séquence nucléotidique présentée dans SEQ ID NO:2 ou un fragment de gène codant un peptide comprenant une séquence d'aminoacides partielle de ladite protéine.

9. Procédé selon l'une quelconque des revendications 1 à 8, où ledit transformant est une cellule animale choisie dans le groupe consistant en une cellule d'ovaire de hamster chinois (cellule CHO), une cellule de myélome de souris, une cellule BHK21, une cellule 293 et une cellule COS.
